# EUROPEAN PATENT SPECIFICATION

(11) **EP 3 324 920 B1**
(45) Date of publication and mention of the grant of the patent: **02.12.2020**
(21) Application number: 16766357.4
(22) Date of filing: 15.07.2016
(51) Int. Cl.: A61K 8/25, A61K 8/36, A61K 8/44, A61Q 1/10, A61K 8/81, A61K 8/86, A61K 8/02, A61K 8/49

(54) **COSMETIC COMPOSITION IN THE FORM OF A COMPACT POWDER, MANUFACTURING PROCESS AND USES THEREOF**
KOSMETISCHE ZUSAMMENSETZUNG IN FORM EINES KOMPAKTEN PUDERS, HERSTELLUNGSVERFAHREN UND VERWENDUNGEN DAVON
COMPOSITION COSMÉTIQUE SE PRÉSENTANT SOUS LA FORME D'UNE POUDRE COMPACTE, PROCÉDÉ DE FABRICATION ET UTILISATIONS DE CELLE-CI

(30) Priority: 24.07.2015 IT UB20152471
(43) Date of publication of application: 30.05.2018
(73) Proprietor: B. Kolormakeup & Skincare S.p.A., 24047 Treviglio (BG) (IT)
(72) Inventor: DE LUIGI, Mario, 20129 Milano (IT)
(74) Representative: Marturano, Pasqualino
(86) International application number: PCT/IB2016/054241
(87) International publication number: WO 2017/017552

(56) References cited:
- WO-A1-2010/109545
- WO-A1-2014/149467
- WO-A2-2014/086710
- FR-A1- 2 865 385
- US-A- 5 851 277
- US-A1- 2011 059 143
- US-A1- 2014 044 660
- Anonymous: "GNPD - Eye Divine Soft Shimmer Shadow", , 1 December 2014 (2014-12-01), XP055255916, Retrieved from the Internet: URL:http://www.gnpd.com/sinatra/recordpage /2846225/from_search/ZA1GtTIPun/ [retrieved on 2016-03-07]

## Description

The present invention relates to a cosmetic composition in the form of a compact powder, its preparation process and relative uses.

Products in the form of a compact powder are an important group of cosmetic products with numerous applications. In particular, these products are widely used for the decoration and cosmetic care of the skin of the face or body. Cosmetic products in the form of a compact powder include, for example, eyeshadows, face powders, compact foundations, blushes, compact mascaras, earth compact powders and the like.

In the state of the art, cosmetic products in the form of a compact powder are obtained by pressing a mixture of ingredients in powder form inside a container made of glass, metal or polymeric material (so-called pan or godet).

Unlike free cosmetic powders, products in compact powder form offer the advantage of being easier to use, easier to transport and having a reduced encumbrance.

Products in compact powder form, however, have the disadvantage of requiring a specific applicator (for example, a brush or sponge) for allowing an adequate collection of the powder from the container and its correct application on the skin.

These products, moreover, are relatively fragile and can therefore easily break following even small impacts of the container.

Furthermore, as the powder with which the above products are formed must be easily picked up in the dry state for application on the skin, these products can easily cause undesired dispersions of the same outside the container.

Cosmetic products in compact powder form are also known in the state of the art, in particular eyeshadows, which can be picked up and distributed on the skin using both a dry applicator, in this case being used as conventional powders, and also using a wet applicator, in this case being used as a foundation. These products, also known as *"two-way cakes"* or *"wet & dry"* products are generally composed of 75-99% by weight of powder components and 1-25% by weight of one or more binders, for example oils, that guarantee the cohesion of the powders in compact form (cake). In order to allow the powders of the products of the *"two-way cake"* type to also be picked up when wet, they must be composed of substantially hydrophobic compounds. Otherwise, indeed, the water present on the wet applicator would be incorporated in the product and the applicator would remain attached to the latter, preventing the correct picking up of the portion of product to be applied. Cosmetic products of the *"two-way cake"* type are described, for example, in US 6,395,301 B1.

The main goal of the present invention is to overcome the drawbacks of the cosmetic compositions in compact powder form of the state of the art.

Within this overall goal, a first objective of the present invention is to provide a composition in the form of a compact powder, suitable, in particular, for the decoration and/or cosmetic care of the skin, which can be applied by putting it directly in contact with the skin, without resorting to the use of an applicator.

A second objective of the present invention is to provide a cosmetic composition in the form of a compact powder that is self-supporting, i.e. that is capable of maintaining its form without the aid of external means, so that it can also be used without pans or other containment means.

A third objective of the present invention is to provide a cosmetic composition in the form of a compact powder that has a higher impact resistance compared to the products of the prior art.

A fourth objective of the present invention is to provide a cosmetic composition in the form of a compact powder that has a low tendency to the accidental release of powder in the surrounding environment.

The Applicant has now found that the above and other objectives that will appear more evident in the following of the present description, can be achieved, according to a first aspect of the present invention, by means of a cosmetic composition in the form of a compact powder comprising (weight percentages referring to the weight of the cosmetic composition):
(a) 30-90% of at least one powder filler,
(b) 3-30% of at least one fatty phase having a melting point equal to or higher than 30°C,
(c) 1-20% of at least one water-soluble polymer,
(d) 3-30% of at least one surfactant, that is liquid at a temperature of 25° C as defined in claim 1 and
(e) less than 5 % of water.

According to a second aspect, the present invention relates to a method for decorating and/or cosmetically treating a keratin surface (e.g. skin) which comprises the following steps:
wetting at least one surface of the above cosmetic composition in the form of a compact powder, so as to form, on said surface of the above cosmetic composition, at least one fluid phase wherein at least a fraction of the powders forming the above cosmetic composition is dispersed;
applying said fluid phase to at least one keratin surface.

According to a third aspect, the present invention relates to a process for preparing the above cosmetic composition in the form of a compact powder that comprises:
- mixing at least the following components:
   (a) 30-90% of at least one powder filler,
   (b) 3-30% of at least one fatty phase having a melting point equal to or higher than 30°C,
   (c) 1-20% of at least one water-soluble polymer,
   (d) 3-30% of at least one surfactant, which is liquid at a temperature of 25° C as defined in claim 1 and (e) less than 5 % of water to form a powdery mixture;
- pressing said powdery mixture to form said cosmetic composition in the form of a compact powder.

Further characteristics and advantages of the present invention will appear more evident from the following description.

The Applicant has surprisingly found that by pressing a mixture of powders for cosmetic use which includes at least one water-soluble polymer and the at least one surfactant, which is liquid at room temperature (25°C), in certain weight ratios, a solid compact composition can be obtained, that can be used for decorating and/or cosmetically treating the skin by putting the above composition directly in contact with the skin, after hydrating the surface of said composition. The cosmetic composition can then be advantageously applied, also without resorting to the use of an applicator.

In a preferred embodiment, the product is in the form of a self-supporting compact powder (for example, a tablet), that does not require containment or supporting means.

The composition in the form of a compact powder according to the present invention is prepared in a substantially anhydrous form. Before use, at least a surface of the composition is hydrated by the addition of a small quantity of water (hydration water). Thanks to the combined action of the water-soluble polymer and the liquid surfactant, the hydration water forms, on the surface of the composition, a thin layer of a fluid phase inside which a fraction of the powders forming the composition remains dispersed.

The fluid phase containing the dispersed powders can be easily transferred to the surface of the skin by simple contact with the same, for example by rubbing the composition onto the skin so that the fluid phase comes into contact with the surface to be treated.

The deposition of the fluid phase onto the skin can also be effected by means of an applicator, such as a brush or a sponge of the type used for applying the compositions in the form of compact powder of the known art, to the skin.

The composition according to the present invention therefore offers the advantage of being usable for decorating and/or cosmetically treating the skin in two ways, i.e.: (i) applying it in a conventional way by means of an applicator; (ii) by applying it, more advantageously, without the aid of an applicator, by putting the composition in direct contact with the skin.

As the composition according to the present invention can be obtained in a self-supporting form, it can also be easily managed by the user and easily packaged in an industrial production cycle.

The composition of the present invention, moreover, has a high compactness and a smooth surface and cannot be substantially picked up in dry form, i.e. in the absence of hydration. The composition consequently does not dirty the skin or other surfaces with which it may come into contact, unless it has been preliminarily hydrated.

The characteristics of compactness of the composition according to the present invention also prevent the accidental dispersion of powders from the surface of the composition. The high compactness, moreover, makes the composition particularly robust and impact-resistant.

The composition can therefore be easily transported, without giving rise to accidental spills or being subject to breakage as a result of impacts.

The cosmetic composition according to the present invention can be advantageously used for the decoration and/or cosmetic treatment of keratin surfaces, such as human or animal skin. The keratin surfaces that can be treated with the cosmetic composition according to the present invention include facial skin, body skin, hair, eyelashes and lips.

In particular, the present invention can be advantageously used for the preparation of cosmetic compositions such as, for example, eyeshadows, face powders, foundation and blushes.

The cosmetic composition according to the present invention can also be advantageously used as a carrier for depositing an active substance, such as, for example, a pharmaceutically or dermatologically active compound, on the above keratin surfaces.

The composition according to the present invention is in the form of a compact powder. The above composition is preferably in the form of a self-supporting compact powder, such as, for example, a pellet, a tablet, or a bar. The self-supporting compact composition can be produced in a wide variety of forms and dimensions. In particular, the composition can have such a form as to be able to be easily held or gripped by the user during application.

For the purposes of the present invention, the term "self-supporting" means that the composition according to the present invention, at room temperature, is capable of sustaining its own weight without undergoing deformation, without requiring containment means, such as for example, casings, capsules or pans.

Although the composition according to the present invention can be advantageously prepared in self-supporting form, it can, however, be conventionally packaged, for example by compacting the formulation in a container for cosmetic use, such as a pan of the type known in the state of the art.

According to the present invention, the cosmetic composition in the form of a compact powder is substantially anhydrous.

The limits and numerical ranges expressed in the present description and annexed claims also include the numerical value(s) indicated. Furthermore, all the values and sub-ranges of a limit or numerical range should be considered as being specifically included as if they had been specifically mentioned.

For the purposes of the present invention, the term "substantially anhydrous composition" means that the water content of the composition in compact powder form is less than 5% by weight with respect to the weight of the composition, preferably less than 3% by weight.

The composition according to the present invention comprises at least one powder filler.

For the purposes of the present invention, the filler includes white or coloured particles, inorganic or organic, lamellar or non-lamellar, having the function of giving body, rigidity or softness to the composition.

The particles of filler preferably have an average diameter within the range of 2-40 micrometers (expressed as dᵥ₅₀, measurable according to ISO 13320:2009).

Examples of powder fillers that can be used for the purposes of the present invention are: inorganic silicates (e.g. micas, talc, silica, kaolin), zinc oxide, titanium oxide, zirconium oxide, cerium oxide, calcium carbonate, magnesium carbonate, boron nitride and bismuth oxychloride.

The micas can be selected, for example, from: muscovite, phlogopite, tiotite, sericite, lepidolite, paragonite and synthetic micas.

Synthetic fillers can also be used, such as Nylon® powders, Teflon® powders, polymethylmethacrylate powders, polyurethane powders, hollow microspheres.

The powder filler is preferably present in the composition in a quantity within the range of 30-90% by weight with respect to the weight of components (a), (b), (c) and (d), more preferably within the range of 50-85% by weight. The combination of components (a), (b), (c) and (d) is also indicated hereunder as "base composition".

The cosmetic composition according to the present invention can also include a mixture of two or more of the above fillers.

The cosmetic composition according to the present invention comprises at least one water-soluble polymer, whose main function is to absorb the water used for hydrating the composition (also indicated herein as "hydration water") before use.

It is believed, that owing to its affinity towards the polymer, the hydration water acts on the surface of the composition in compact powder form, partially crumbling the latter and forming a fluid phase in which a fraction of the powders forming the composition is dispersed. The presence of the water-soluble polymer in the fluid phase, moreover, favours the distribution and adhesion of the powders on the surface of the skin.

For the purposes of the present invention, a polymer is considered as being "water-soluble" if it dissolves in water, at room temperature (i.e. 25°C) and atmospheric pressure, in a quantity equal to or higher than 5% by weight, preferably equal to or higher than 10% by weight.

The water-soluble polymer is preferably selected from: polyvinylpyrrolidone (PVP), vinylpyrrolidone-vinyl acetate copolymer, polyvinyl alcohol (PVA), and mixtures thereof.

More preferably the water-soluble polymer is selected from: polyvinylpyrrolidone, vinylpyrrolidone-vinyl acetate copolymer and mixtures thereof.

The polyvinylpyrrolidone and vinylpyrrolidone-vinyl acetate copolymer preferably have an average molecular weight (weight average M_{W} determined by GPC) within the range of 10.000 - 100.000 g/mol, more preferably within the range of 20.000 - 50.000 g/mol.

The water-soluble polymer is preferably present in the composition in a quantity within the range of 1-20% by weight with respect to the weight of the base composition, more preferably within the range of 3-12% by weight.

The cosmetic composition according to the present invention also comprises at least one fatty phase acting as binder. The fatty phase is composed of fatty substances which facilitate the cohesion of the powdery compounds between each other in the compacted composition and also their adhesion to the skin.

The fatty phase has a melting point at atmospheric pressure which is equal to or higher than 30°C, preferably within the range of 30-250°C, more preferably within the range of 30-230°C.

The fatty substances that form the fatty phase are preferably selected from: waxes, metal soaps of C₈-C₂₂ fatty acids, preferably C₁₂-C₁₈ fatty acids, and mixtures thereof.

For the purposes of the present invention, the term "wax" means a lipophilic fatty compound which is solid at room temperature (i.e. 25°C) and atmospheric pressure and which shows a reversible change of the solid/liquid state; in particular, the wax has a melting point equal to or higher than 30°C, preferably equal to or higher than 55°C, and equal to or lower than 250°C, preferably equal to or lower than 230°C, more preferably lower than 120°C. The above melting points can be determined, for example, by means of differential scanning calorimetry (DSC), with a temperature increase equal to 5°C/minute.

Examples of waxes that can be used for the purposes of the present invention are: beeswax, lanolin, Carnauba wax, paraffin waxes, polyethylene waxes, silicone or fluorinated waxes and mixtures thereof.

The metal soaps of fatty acids preferably used for the purposes of the present invention are zinc or magnesium soaps.

The C₈-C₂₂ fatty acids are preferably selected from lauric acid, myristic acid, stearic acid, palmitic acid and mixtures thereof.

Preferred examples of metal soaps that can be used for the purposes of the present invention are: magnesium stearate, zinc laurate, magnesium myristate, zinc stearate and mixtures thereof, preferably magnesium stearate. A particularly preferred fatty phase is magnesium stearate.

The fatty phase is preferably present in the composition in a quantity within the range of 3-30% by weight with respect to the weight of the base composition, more preferably within the range of 5-30% by weight.

The cosmetic composition according to the present invention also comprises at least one surfactant which is liquid at 25°C and atmospheric pressure, as defined in claim 1.

The above surfactant preferably has, at atmospheric pressure, a melting point equal to or lower than 30°C and preferably equal to or higher than 0°C.

For the purposes of the present invention, a surfactant has a melting point equal to or lower than 30°C and equal to or higher than 0°C, at atmospheric pressure, if, under said temperature and pressure conditions, said surfactant is at least partly in the liquid state.

The surfactant has the function of favouring the formation of the fluid phase on the surface of the compacted composition, following the addition of the hydration water. The surfactant also acts as an emulsifier favouring the dispersion of the powders in the fluid phase and the stability of the latter once formed. With respect to surfactants in the solid state, the use of surfactants in the liquid state at room temperature makes the formation of the fluid phase simpler and more rapid and gives a mere pleasant sensation on the user's skin.

The composition can also comprise a mixture of two or more surfactants.

The overall quantity of surfactants present in the composition is preferably within the range of 3-30% by weight referring to the weight of the base composition, more preferably within the range of 5-20% by weight, even more preferably within the range of 7-20% by weight.

The non-ionic surfactants are esters of N-acylated amino acids having the following formula (I):

R₁(CO)N(R₂)CH(R₃)(CH₂)ₙ(CO)OR₄ (I)

wherein:
n is 0 or an integer equal to 1 or 2,
R₁ represents a linear or branched C₅-C₂₁ alkyl radical, or a linear or branched C₅-C₂₁ alkenyl radical,
R₂ represents a hydrogen atom or C₁-C₃ alkyl group,
R₃ represents a C₁-C₄ alkyl radical,
R₄ represents a C₁-C₁₀ alkyl radical, a linear or branched C₂-C₁₀ alkenyl radical, or a sterol group, and
the N(R₂)CH(R₃)(CH₂)ₙ(CO) part is selected from the following amino acids: valine, leucine, isoleucine, serine, threonine, proline, hydroxyproline, β-alanine, aminobutyric acid, amino caproic acid, sarcosine or N-methyl-β-alanine, preferably sarcosine.

The R₁(CO)- group is preferably an acyl group of an acid preferably selected from caproic acid, lauric acid, myristic acid, palmitic acid, stearic acid, behenic acid, linoleic acid, linolenic acid, oleic acid, isostearic acid, 2-ethylhexanoic acid, fatty acids of coconut oil and fatty acids of palm oil.

The above acid is preferably lauric acid.

The N(R₂)CH(R₃)(CH₂)ₙ(CO) part is preferably sarcosine.

The-OR₄ group is preferably obtained from the following alcohols: methanol, ethanol, propanol, isopropanol, butanol, tert-butanol, isobutanol, 3-methyl-1-butanol, 2-methyl-1 butanol, fusel oil, pentanol, hexanol, cyclohexanol, octanol, 2-ethylhexanol, decanol, lauryl alcohol, myristyl alcohol, cetyl alcohol, cetearyl alcohol, stearyl alcohol, oleic alcohol, behenyl alcohol, jojoba alcohol, 2-hexadecyl alcohol, 2-octyldodecanol alcohol and isostearyl alcohol. The alcohol is preferably isopropanol.

The above esters of N-acylated amino acids can be prepared according to techniques known to the person skilled in the art; they can be obtained, for example, from natural amino acid sources. The amino acids can be obtained, for example, by the hydrolysis of natural vegetable proteins (e.g. oats, corn, soybean, palm, coconut) with the formation of mixtures of amino acids; the amino acids thus obtained are then esterified and then N-acylated. Further information on the preparation methods of the above esters of N-acylated amino acids can be found in EP 1072251 A1 and WO 2012/080994.

The cosmetic composition comprises at least one non-ionic surfactant having formula (I), more preferably combined with at least a second surfactant different from said non-ionic surfactant having formula (I). The surfactant having formula (I) is more preferably isopropyl N-lauroylsarcosinate, available on the market, for example, under the tradename "Eldew SL-205" (Ajinomoto).

Non-ionic surfactants having formula (I), in particular isopropyl N-lauroylsarcosinate, favour the formation of the fluid phase following the addition of the hydration water, as they act as emulsifiers of the fatty phase and of the cosmetic powders present in the composition.

Examples of anionic surfactants that can be used in the present invention are: polyalkylene glycol ethers of fatty alcohols, taurates, acyl lactylates, sodium stearoyl lactylate, alkyl sulfates, sodium lauryl sulfate, polyoxyethylenated alkyl sulfates, alkyl ether sulfate, monoethanolamine lauryl ether sulfate, carboxylated alkyl ethers, monoalkyl phosphate, dialkyl phosphate, arginine mono-(2-hexyldecyl) phosphate, ethoxylated alkyl phosphate, N-acyl sarcosinate, sodium lauryl sarcosinate, sodium myristoyl sarcosinate, N-acylglutamate, sodium lauroyl glutamate, acetyl isethionate, sodium cocoyl isethionate, potassium laurate, potassium myristate, potassium palmitate, potassium stearate and mixtures thereof.

Examples of cationic surfactants that can be used in the present invention are: quaternary ammonium salts, in particular ammonium halides, alkyltrimethylammonium halides, dialkyl-dimethylammonium halides, trialkyl-methylammonium halides; carboxylated derivatives of pyrrolidone such as PCA ethyl cocoyl arginate and the salt ethyl-N-cocoyl-L-arginate of D, L-2-pyrrolidone-5-carboxylic acid.

Examples of amphoteric surfactants that can be used in the present invention are: betaines and derivatives, sulfobetaine and derivatives, imidazole derivatives, such as sodium cocoamphodiacetate.

The composition according to the present invention can also comprise one or more pigments.

For the purposes of the present invention, the term "pigment" includes white or coloured particles, inorganic or organic, having the function of colouring and/or opacifying the composition, on the condition that in each specific cosmetic composition, the pigment is different from the filler.

The pigments can be selected from organic pigments and mineral pigments known in the state of the art. The pigments are preferably used in the compositions in the form of powder or paste. The pigments can be coated or uncoated.

The pigments can be selected, for example, from natural pigments (minerals), organic pigments, lacquers, pigments with particular effects, such as pearlescent pigments ("nacres"), and mixtures thereof.

For the purposes of the present invention, the term "lacquers" refers to dyes made insoluble by adsorption on insoluble particles (e.g. particles of alumina, silica, calcium and sodium borosilicate, calcium and aluminium borosilicate); the compounds thus obtained remain substantially insoluble during the use of the composition.

The natural pigments can be selected, for example, from iron oxides, micas, chromium oxides, titanium dioxide, manganese violet, ultramarine blue.

The organic pigments can be selected, for example, from: nitroso compounds, nitro compounds, azo compounds, xanthane compounds, quinoline compounds, anthraquinone compounds, phthalocyanine compounds, metal complexes, isoindolinone compounds, quinacridone compounds, perinone compounds, perylene compounds, thioindigo compounds.

Examples of pearlescent pigments are for example: particles of mica coated with titanium or coated with bismuth oxychloride or coated with iron oxides, etc.

The pigments are preferably present in the composition in an overall quantity within the range of 1-50% by weight with respect to the weight of the base composition, more preferably within the range of 2-40% by weight.

The composition according to the present invention can also comprise one or more further ingredients (additives), in particular additives conventionally used in cosmetic compositions, especially compositions for decoration and/or skin care.

The additives are preferably incorporated in the composition in the solid state in the form of particles. Liquid additives, before being mixed with the other ingredients of the composition, can be absorbed on a solid ingredient, for example, a filler.

Examples of additives that can be used are: fragrances, preservatives, antioxidants, rheology modifiers, pH regulators, sequestering agents, emollients, cosmetic or dermatological active ingredients (e.g. vitamins, anti-inflammatory agents, etc.) and mixtures thereof.

The additives are preferably present in the composition in an overall quantity within the range of 0.01-20% by weight with respect to the weight of the base composition, more preferably within the range of 0.05-10% by weight.

The cosmetic composition according to the present invention can be prepared according to techniques and equipment known to the person skilled in the art.

The composition in powder form can be obtained by mixing the powders of the various components together, for example in a mixer or in a mill, preferably at room temperature.

In a preferred embodiment, the preparation process envisages the preliminary mixing of the solid components, so as to form a powdery pre-mixture. The liquid components are then incorporated in the pre-mixture, homogenizing them with the latter until a final homogeneous mixture is obtained.

Each of the above mixing and homogenization phases can last, for example, from 1 to 60 minutes.

Before compacting, the final mixture can be subjected to sieving. The sieving can be effected, for example, with 25 to 90 mesh sieves.

The final mixture in powder form, after possible sieving, can be pressed to obtain the composition in compact powder form according to the invention.

The compacting can be effected, for example, with a conventional tablet press. The compacting is preferably carried out at a pressure within the range of 5-200 bar, more preferably 30-100 bar.

The mixing and compacting phases are preferably carried out at room temperature.

The composition according to the present invention can also be prepared in compact form inside a pan. For this purpose, after pouring the final mixture into a pan or other suitable container, it is subsequently pressed. For this purpose, the same compacting devices can be used, as those adopted for the preparation of the products in compact powder form inside pans of the state of the art.

In order to deposit and distribute the composition according to the present invention on the skin, the compacted composition is hydrated on the surface. The hydration is preferably effected by pouring a small quantity of water (for example, a few drops) onto at least a surface of the composition.

The water acts on the surface layer of the composition, causing its partial crumbling with the formation of a thin layer of a fluid phase in which the powders of the composition are dispersed.

The fluid phase can be picked up from the surface of the composition with a finger, for example, or with an applicator of the type known in the state of the art (e.g. a brush or sponge). The fluid phase can then be transferred onto the skin with a finger or with the applicator.

The fluid phase can also be obtained by rubbing a wet finger or a wet applicator on the surface of the dry composition and can be transferred to the skin with the same means.

In a particularly advantageous embodiment of use, the fluid phase can be transferred from the surface of the composition to the surface of the skin by rubbing the composition onto the skin so that the fluid phase comes into direct contact with the skin, thus avoiding the use of an applicator. This mode of use is particularly advantageous when the composition is in self-supporting form.

The fluid phase obtained as described above easily and firmly adheres to the skin.

The application of the wet cosmetic composition offers the advantage of allowing decorative effects having a greater aesthetic quality to be obtained with respect to the application of dry powders, in particular allowing shaded decorative effects to be obtained.

By varying the quantity of hydration water, moreover, more or less diluted fluid phases and consequently different decorative effects can be obtained, according to the user's requirements.

Furthermore, unlike the dry application of powders, the application of wet make-up according to the present invention gives the user a pleasant sensation of freshness.

The following embodiment example is provided for purely illustrative and should not be intended as limiting the protection scope defined by the enclosed claims.

### EXAMPLE

A cosmetic composition was prepared according to the present invention, that can be used as eyeshadow for the face having the following weight percentage composition (base composition):

| | | |
|---|---|---|
| (a) | micronized talc (average diameter of the powders (dv50): 10 µm) | 72.7% |
| (b) | magnesium stearate | 11.4% |
| (c) | PVP K-30 (Ashland) | 5.7% |
| (d1) | polysorbate-20 (non-ionic surfactant, HLB about 17, TEGO®-SML-20 (Degussa)) | 6.8% |
| (d2) | isopropyl N-lauroylsarcosinate (non-ionic surfactant, ELDEW SL-205 (Ajinomoto)) | 3.4% |

The composition also contained a mixture of pigments in powder form (CI77510, CI77742, CI77007) in an overall quantity of 9.5% by weight with respect to the weight of the base composition, and preservatives in a quantity equal to 4.1% by weight with respect to the weight of the base composition.

The composition was prepared by mixing the solid components in a mixing mill at about 3,300 rpm for 2 minutes. At the end of the mixing, the liquid components, i.e. the two non-ionic surfactants, were introduced into the mixing mill. The mixing was restarted and prolonged for 4 minutes.

The composition was then discharged from the mixing mill and sieved in screens having 25 mesh sieves. The sieved powder was compacted into disc-shaped tablets (1.7 g each) using a hydraulic tablet press and applying a pressure of 40 bar.

The tablets were subjected to the drop test to measure the cohesion of the composition.

The drop test was effected on 10 tablets (diameter 18 mm, height 5 mm) as follows. Each tablet was dropped from a height of 30 cm onto a steel surface. The operation was then repeated until the breakage of the tablet after being dropped.

For the purposes of the present invention, the cohesion degree of the composition is considered sufficient (drop test passed) if the tablet resists at least three consecutive drops without breaking.

In the case of the tablets according to the present invention, breakage was observed on average after the 6^{th}-7^{th} drop.

## Claims

1. Cosmetic composition in the form of a compact powder comprising (percentages by weight based on the weight of the cosmetic composition):
(a) 30-90% of at least one powder filler,
(b) 3-30% of at least one fatty phase having a melting point equal to or greater than 30°C,
(c) 1-20% of at least one water-soluble polymer that dissolves in water, at 25°C and atmospheric pressure, in a quantity equal to or higher than 5% by weight,
(d) 3-30% of at least one surfactant that is liquid at the temperature of 25°C;
(e) less than 5% of water;
wherein said at least one liquid surfactant (d) is an N-acylated amino acid of formula (I)
R₁(CO)N(R₂)CH(R₃)(CH₂)ₙ(CO)OR₄ (I)
wherein:
n is 0 or an integer equal to 1 or 2,
R₁ represents a linear or branched C₅-C₂₁ alkyl radical, or a linear or branched C₅-C₂₁ alkenyl radical,
R₂ represents a hydrogen atom or C₁-C₃ alkyl group,
R₃ represents a C₁-C₄ alkyl radical,
R₄ represents a C₁-C₁₀ alkyl radical, a linear or branched C₂-C₁₀ alkenyl radical, or a sterol group, and
the N(R₂)CH(R₃)(CH₂)ₙ(CO) portion is selected from the following amino acids: valine, leucine, isoleucine, serine, threonine, proline, hydroxyproline, β-alanine, aminobutyric acid, amino caproic acid, sarcosine or N-methyl-β-alanine.

2. The composition according to the previous claim, wherein said at least one liquid surfactant has a melting point equal to or higher than 0°C.

3. Composition according to claim 1 or 2, wherein said solid fatty phase is selected from: wax, metal soap of a C₈-C₂₂ fatty acid or mixtures thereof.

4. Composition according to the preceding claim, wherein said metal soap of a C₈-C₂₂ fatty acid is selected from: zinc stearate, magnesium stearate, zinc laurate, magnesium laurate, zinc myristate, magnesium myristate or mixtures thereof.

5. Composition according to any one of the preceding claims, wherein said water soluble polymer is selected from: polyvinylpyrrolidone, vinylpyrrolidone-vinyl acetate copolymer, polyvinyl alcohol, or mixtures thereof, preferably polyvinylpyrrolidone, vinylpyrrolidone-vinyl acetate copolymer or mixtures thereof.

6. Composition according to the preceding claim, wherein said water soluble polymer is selected from polyvinylpyrrolidone, vinylpyrrolidone-vinyl acetate copolymer or mixtures thereof having an average molecular weight (weight average M_{W}) in the range 10.000 - 100.000 g/mol, preferably in the range 20.000 - 50.000.

7. Composition according to claim 1, wherein said surfactant of formula (I) is isopropyl N-lauroyl sarcosinate.

8. Composition according to claim 1 comprising at least one second surfactant different from said surfactant having formula (I).

9. Composition according to any one of the preceding claims which comprises at least one pigment, preferably in an amount in the range of 1%-50% by weight with respect to the overall weight of said components (a), (b), (c) and (d).

10. Method for decorating and/or cosmetically caring for a keratin surface which comprises the steps of:
(a) wetting at least one surface of a cosmetic composition according to claim 1 so as to form, on said surface of said cosmetic composition, at least one fluid phase wherein at least one fraction of the powders forming said cosmetic composition is dispersed therein;
(b) applying said fluid phase to at least one keratin surface.

11. Method according to the preceding claim wherein said application step (b) comprises rubbing said cosmetic composition on said keratin surface so as to put said wetted surface comprising said fluid phase in contact with said keratin surface.

12. Method according to claim 10 wherein said fluid phase is drawn from said wetted surface by means of an applicator and applied to said keratin surface by means of said applicator.

13. Process for preparing a cosmetic composition in the form of a compact powder according to claim 1 that comprises (percentages by weight based on the weight of the cosmetic composition):
- mixing at least the following components:
(a) 30-90% of at least one powder filler,
(b) 3-30% of at least one solid fatty phase having a melting point equal to or greater than 30°C,
(c) 1-20% of at least one water soluble polymer that dissolves in water, at 25°C and atmospheric pressure, in a quantity equal to or higher than 5% by weight,
(d) 3-30% of at least one surfactant that is liquid at the temperature of 25°C said surfactant being an N-acylated amino acid of formula (I)
R₁(CO)N(R₂)CH(R₃)(CH₂)ₙ(CO)OR₄ (I)
wherein:
n is 0 or an integer equal to 1 or 2,
R₁ represents a linear or branched C₅-C₂₁ alkyl radical, or a linear or branched C₅-C₂₁ alkenyl radical,
R₂ represents a hydrogen atom or C₁-C₃ alkyl group,
R₃ represents a C₁-C₄ alkyl radical,
R₄ represents a C₁-C₁₀ alkyl radical, a linear or branched C₂-C₁₀ alkenyl radical, or a sterol group, and
the N(R₂)CH(R₃)(CH₂)ₙ(CO) portion is selected from the following amino acids: valine, leucine, isoleucine, serine, threonine, proline, hydroxyproline, β-alanine, aminobutyric acid, amino caproic acid, sarcosine or N-methyl-β-alanine,
to form a powdery mixture;
- pressing said powdery mixture to form said cosmetic composition in the form of a compact powder having a water content lower than 5%.

14. Device comprising at least one solid cosmetic composition according to claim 1 and at least one applicator.

## Patentansprüche

1. Kosmetische Zusammensetzung in Form eines kompakten Puders, Folgendes umfassend (Gewichtsprozente bezogen auf das Gewicht der kosmetischen Zusammensetzung):
(a) 30-90 % mindestens eines puderförmigen Füllstoffs,
(b) 3-30 % mindestens einer Fettphase, die einen Schmelzpunkt gleich oder höher als 30 °C aufweist,
(c) 1-20 % mindestens eines wasserlöslichen Polymers, das sich bei 25 °C und Luftdruck in einer Menge von 5 Gew.-% oder mehr in Wasser löst,
(d) 3-30 % mindestens eines Tensids, das bei einer Temperatur von 25 °C flüssig ist;
(e) weniger als 5 % Wasser;
wobei das mindestens eine flüssige Tensid (d) eine N-acylierte Aminosäure der Formel (I) ist
R₁(CO)N(R₂)CH(R₃)(CH₂)ₙ(CO)OR₄ (I)
wobei:
n 0 oder eine ganze Zahl gleich 1 oder 2 ist,
R₁ einen geradkettigen oder verzweigten C₅-C₂₁-Alkylrest oder einen geradkettigen oder verzweigten C₅-C₂₁-Alkenylrest darstellt,
R₂ ein Wasserstoffatom oder eine C₁-C₃-Alkylgruppe darstellt,
R₃ einen C₁-C₄-Alkylrest darstellt,
R₄ einen C₁-C₁₀-Alkylrest, einen geradkettigen oder verzweigten C₂-C₁₀-Alkenylrest oder eine Sterolgruppe darstellt, und
der N(R₂)CH(R₃)(CH₂)ₙ(CO)-Anteil aus den folgenden Aminosäuren ausgewählt ist: Valin, Leucin, Isoleucin, Serin, Threonin, Prolin, Hydroxyprolin, β-Alanin, Aminobuttersäure, Aminocapronsäure, Sarkosin oder N-Methyl-β-alanin.

2. Zusammensetzung gemäß dem vorhergehenden Anspruch, wobei das mindestens eine flüssige Tensid einen Schmelzpunkt gleich oder höher als 0 °C aufweist.

3. Zusammensetzung gemäß Anspruch 1 oder 2, wobei die feste Fettphase aus Folgendem ausgewählt wird: Wachs, Metallseife einer C₈-C₂₂-Fettsäure oder Gemischen davon.

4. Zusammensetzung gemäß dem vorhergehenden Anspruch, wobei die Metallseife einer C₈-C₂₂-Fettsäure aus Folgendem ausgewählt wird: Zinkstearat, Magnesiumstearat, Zinklaurat, Magnesiumlaurat, Zinkmyristat, Magnesiummyristat oder Gemischen davon.

5. Zusammensetzung gemäß einem der vorhergehenden Ansprüche, wobei das wasserlösliche Polymer aus Folgendem ausgewählt wird: Polyvinylpyrrolidon, Vinylpyrrolidon-Vinylacetat-Copolymer, Polyvinylalkohol oder Gemischen davon, vorzugsweise Polyvinylpyrrolidon, Vinylpyrrolidon-Vinylacetat-Copolymer oder Gemischen davon.

6. Zusammensetzung gemäß dem vorhergehenden Anspruch, wobei das wasserlösliche Polymer aus Folgendem ausgewählt wird: Polyvinylpyrrolidon, Vinylpyrrolidon-Vinylacetat-Copolymer oder Gemischen davon, aufweisend ein mittleres Molekulargewicht (Gewichtsmittel Mw) im Bereich von 10.000 - 100.000 g/mol, vorzugsweise im Bereich von 20.000 - 50.000.

7. Zusammensetzung gemäß Anspruch 1, wobei das Tensid der Formel (I) Isopropyl-N-lauroylsarkosinat ist.

8. Zusammensetzung gemäß Anspruch 1, umfassend mindestens ein zweites Tensid, das sich von dem Tensid unterscheidet, der die Formel (I) aufweist.

9. Zusammensetzung gemäß einem der vorhergehenden Ansprüche, die jeweils mindestens ein Pigment umfasst, vorzugsweise in einer Menge im Bereich von 1 bis 50 Gew.-% bezogen auf das Gesamtgewicht der Bestandteile (a), (b), (c) und (d) .

10. Verfahren zum Dekorieren und/oder zur kosmetischen Pflege einer Keratinoberfläche, welches folgende Schritte umfasst:
(a) Befeuchten mindestens einer Oberfläche einer kosmetischen Zusammensetzung gemäß Anspruch 1, um auf der Oberfläche der kosmetischen Zusammensetzung mindestens eine flüssige Phase zu bilden, wobei mindestens ein Anteil der Puder, die die kosmetische Zusammensetzung bilden, darin dispergiert ist;
(b) Auftragen der flüssigen Phase auf mindestens eine Keratinoberfläche.

11. Verfahren gemäß dem vorhergehenden Anspruch, wobei der Auftragschritt (b) das Reiben der kosmetischen Zusammensetzung auf der Keratinoberfläche umfasst, um die benetzte Oberfläche, die die flüssige Phase umfasst, mit der Keratinoberfläche in Kontakt zu bringen.

12. Verfahren gemäß Anspruch 10, wobei die flüssige Phase mit Hilfe eines Applikators von der benetzten Oberfläche entfernt und mit Hilfe des Applikators auf die Keratinoberfläche aufgetragen wird.

13. Verfahren zur Herstellung einer kosmetischen Zusammensetzung in Form eines kompakten Puders gemäß Anspruch 1, die Folgendes umfasst (Gewichtsprozente bezogen auf das Gewicht der kosmetischen Zusammensetzung):
- Mischen mindestens der folgenden Bestandteile:
(a) 30-90 % mindestens eines puderförmigen Füllstoffs,
(b) 3-30 % mindestens einer festen Fettphase, die einen Schmelzpunkt gleich oder höher als 30 °C aufweist,
(c) 1-20 % mindestens eines wasserlöslichen Polymers, das sich bei 25 °C und Luftdruck in einer Menge von 5 Gew.-% oder mehr in Wasser löst,
(d) 3-30 % mindestens eines Tensids, das bei einer Temperatur von 25 °C flüssig ist, wobei das Tensid eine N-acylierte Aminosäure der Formel (I) ist
R₁(CO)N(R₂)CH(R₃)(CH₂)ₙ(CO)OR₄ (I)
wobei:
n 0 oder eine ganze Zahl gleich 1 oder 2 ist,
R₁ einen geradkettigen oder verzweigten C₅-C₂₁-Alkylrest oder einen geradkettigen oder verzweigten C₅-C₂₁-Alkenylrest darstellt,
R₂ ein Wasserstoffatom oder eine C₁-C₃-Alkylgruppe darstellt,
R₃ einen C₁-C₄-Alkylrest darstellt,
R₄ einen C₁-C₁₀-Alkylrest, einen geradkettigen oder verzweigten C₂-C₁₀-Alkenylrest oder eine Sterolgruppe darstellt, und
der N(R₂)CH(R₃)(CH₂)ₙ(CO)-Anteil aus den folgenden Aminosäuren ausgewählt ist: Valin, Leucin, Isoleucin, Serin, Threonin, Prolin, Hydroxyprolin, β-Alanin, Aminobuttersäure, Aminocapronsäure, Sarkosin oder N-Methyl-β-alanin,
um ein puderiges Gemisch zu bilden;
- Pressen des puderigen Gemisches, um die kosmetische Zusammensetzung in Form eines kompakten Puders zu bilden, das einen Wassergehalt von weniger als 5 % aufweist.

14. Vorrichtung, die mindestens eine feste kosmetische Zusammensetzung gemäß Anspruch 1 und mindestens einen Applikator umfasst.

## Revendications

1. Composition cosmétique sous forme d'une poudre compacte comprenant (pourcentages en poids sur la base du poids de la composition cosmétique) :
(a) de 30 à 90% d'au moins une charge de poudre,
(b) de 3 à 30% d'au moins une phase grasse ayant un point de fusion égal ou supérieur à 30°C,
(c) de 1 à 20% d'au moins un polymère soluble dans l'eau qui se dissout dans l'eau, à 25°C et à pression atmosphérique, dans une quantité égale ou supérieure à 5% en poids,
(d) de 3 à 30% d'au moins un tensioactif qui est liquide à la température de 25°C ;
(e) moins de 5% d'eau ;
où ledit au moins un tensioactif liquide (d) est un acide aminé N-acylé de formule (I)
R₁(CO)N(R₂)CH(R₃)(CH₂)ₙ(CO)OR₄ (I)
où :
n est 0 ou un entier égal à 1 ou 2,
R₁ représente un radical alkyle en C₅-C₂₁ linéaire ou ramifié, ou un radical alcényle en C₅-C₂₁ linéaire ou ramifié,
R₂ représente un atome d'hydrogène ou un groupe alkyle en C₁-C₃,
R₃ représente un radical alkyle en C₁-C₄,
R₄ représente un radical alkyle en C₁-C₁₀, un radical alcényle en C₂-C₁₀ linéaire ou ramifié ou un groupe stérol, et
la portion N(R₂)CH(R₃)(CH₂)n (CO) est choisie parmi les acides aminés suivants : valine, leucine, isoleucine, sérine, thréonine, proline, hydroxyproline, β-alanine, acide aminobutyrique, acide aminacapoïque, sarcosine ou N-méthyl-β-alanine.

2. Composition selon la revendication précédente, où ledit au moins un tensioactif liquide a un point de fusion égal ou supérieur à 0°C.

3. Composition selon la revendication 1 ou 2, où ladite phase grasse solide est choisie parmi : cire, savon métallique d'un acide gras en C₈-C₂₂ ou leurs mélanges.

4. Composition selon la revendication précédente, où ledit savon métallique d'un acide gras en C₈-C₂₂ est choisi parmi : stéarate de zinc, stéarate de magnésium, laurate de zinc, laurate de magnésium, myristate de zinc, myristate de magnésium ou leurs mélanges.

5. Composition selon l'une quelconque des revendications précédentes, où ledit polymère soluble dans l'eau est choisi parmi : polyvinylpyrrolidone, copolymère vinylpyrrolidone-acétate de vinyle, alcool polyvinylique, ou leurs mélanges, de préférence polyvinylpyrrolidone, copolymère vinylpyrrolidone-acétate de vinyle ou leurs mélanges.

6. Composition selon la revendication précédente, où ledit polymère soluble dans l'eau est choisi parmi polyvinylpyrrolidone, copolymère vinylpyrrolidone-acétate de vinyle ou leurs mélanges ayant un poids moléculaire moyen (moyenne en poids Mw) compris entre 10000 et 100000 g/mol, de préférence entre 20000 et 50000.

7. Composition selon la revendication 1, où ledit tensioactif de la formule (I) est N-lauroyl sarcosinate d'isopropyle.

8. Composition selon la revendication 1 comprenant au moins un second tensioactif actif différent dudit tensioactif ayant la formule (I).

9. Composition selon l'une quelconque des revendications précédentes qui comprend au moins un pigment, de préférence dans une quantité comprise entre 1% et 50% en poids par rapport au poids total desdits composants (a), (b), (c) et (d).

10. Procédé de décoration et/ou de soin cosmétique d'une surface kératinique qui comprend les étapes de :
(a) mouillage d'au moins une surface d'une composition cosmétique selon la revendication 1 de manière à former, sur ladite surface de ladite composition cosmétique, au moins une phase fluide où au moins une fraction des poudres formant ladite composition cosmétique est dispersée dans celle-ci ;
(b) application de ladite phase fluide sur au moins une surface kératinique.

11. Procédé selon la revendication précédente où ladite étape d'application (b) comprend la friction de ladite composition cosmétique sur ladite surface kératinique de manière à mettre ladite surface mouillée comprenant ladite phase fluide en contact avec ladite surface kératinique.

12. Procédé selon la revendication 10 où ladite phase fluide est tirée de ladite surface mouillée au moyen d'un applicateur et appliquée à ladite surface kératinique au moyen dudit applicateur.

13. Procédé de préparation d'une composition cosmétique sous forme d'une poudre compacte selon la revendication 1 qui comprend (pourcentages en poids sur la base du poids de la composition cosmétique) :
- le mixage au moins des composants suivants :
(a) de 30 à 90% d'au moins une charge de poudre,
(b) de 3 à 30% d'au moins une phase grasse solide ayant un point de fusion égal ou supérieur à 30°C,
(c) de 1 à 20% d'au moins un polymère soluble dans l'eau qui se dissout dans l'eau, à 25°C et à pression atmosphérique, dans une quantité égale ou supérieure à 5% en poids,
(d) de 3 à 30% d'au moins un tensioactif qui est liquide à la température de 25°C ledit tensioactif étant un acide aminé N-acylé de formule (I)
R₁(CO)N(R₂)CH(R₃)(CH₂)ₙ(CO)OR₄ (I)
où :
n est 0 ou un entier égal à 1 ou 2,
R₁ représente un radical alkyle en C₅-C₂₁ linéaire ou ramifié, ou un radical alcényle en C₅-C₂₁ linéaire ou ramifié,
R₂ représente un atome d'hydrogène ou un groupe alkyle en C₁-C₃,
R₃ représente un radical alkyle en C₁-C₄,
R₄ représente un radical alkyle en C₁-C₁₀, un radical alcényle en C₂-C₁₀ linéaire ou ramifié ou un groupe stérol, et
la portion N(R₂)CH(R₃)(CH₂)ₙ(CO) est choisie dans les acides aminés suivants : valine, leucine, isoleucine, sérine, thréonine, proline, hydroxyproline, β-alanine, acide aminobutyrique, acide aminacapoïque, sarcosine ou N-méthyl-β-alanine,
pour former un mélange poudreux ;
- la pression dudit mélange poudreux pour former ladite composition cosmétique sous forme d'une poudre compacte ayant une teneur en eau inférieure à 5%.

14. Dispositif comprenant au moins une composition cosmétique solide selon la revendication 1 et au moins un applicateur.
